# EUROPEAN PATENT SPECIFICATION

(11) **EP 4 582 067 B1**
(45) Date of publication and mention of the grant of the patent: **09.12.2026**
(21) Application number: 25178038.3
(22) Date of filing: 21.09.2018
(51) Int. Cl.: A61F 5/445

(54) **AN OSTOMY APPLIANCE**
OSTOMIEVORRICHTUNG
APPAREIL DE STOMIE

(30) Priority: 22.09.2017 GB 201715388
(43) Date of publication of application: 09.07.2025
(62) Document number(s) of the earlier application(s) in accordance with Art. 76 EPC: 18778988.8 / 3 684 300
(73) Proprietor: Salts Healthcare Limited, Birmingham, West Midlands B7 4AA (GB)
(72) Inventor: WILLIAMS, Kieran, Birmingham, B7 4AA (GB); TRETHEWAY, Lee, Birmingham, B7 4AA (GB); POWNER, Iain, Birmingham, B7 4AA (GB); ALFARO, Jesus, Birmingham, B7 4AA (GB); ALLEN, Marcus, Birmingham, B7 4AA (GB)
(74) Representative: Forresters IP LLP

(56) References cited:
- WO-A2-2007/030703
- GB-A- 1 596 496
- US-A1- 2009 247 970

## Description

The invention relates to an ostomy appliance for collecting human waste. It should be understood that the invention can be utilised in drainable and non-drainable ostomy appliances.

It is known to provide in a wall of an ostomy appliance ('bag' or 'pouch' as they are commonly known in the art) an aperture to permit waste gases to escape from a waste collecting chamber of the ostomy appliance. This is necessary to prevent the bag expanding too much with gas and potentially from leaking or bursting whilst filled with waste. In some prior art ostomy appliances the aperture is covered by a filter, the purpose of which is to de-odorise the waste gases before they exit to atmosphere. In the known ostomy appliances the aperture and filter are usually positioned in an upper part of the bag above a stoma receiving opening. Such filters are usually, but not always, positioned inside the exterior wall of ostomy appliance so that waste gas must pass through the filter before exiting the bag via the aperture.

Although intermediate walls are often provided in between the exterior walls in order to create a tortuous path for waste gas to travel through in order to get to the filter, thus making it difficult for bodily waste to come into contact with the filter, these walls are quite often unsuccessful in keeping bodily waste away from the filter. In this case, the filter can become blocked during use which causes the appliance to gradually inflate with waste gases with no means of escaping, causing discomfort and/or embarrassment to the user.

WO2007/030703 relates to a gas filter for an ostomy bag vent, has a gas permeable covering layer for gas-tight attachment to an external surface of an ostomy bag, and covers media layer, barrier layers, and adsorbent layer.

The present invention looks to address this problem.

According to a first aspect of the invention, we provide an ostomy appliance as set out in claim 1.

Further features of the first aspect of the invention are set out in claims 2 and 5 to 15 appended hereto.

According to a second aspect of the invention, the present invention relates to an ostomy appliance as set out in claim 3.

Further features of the second aspect of the invention are set out in claims 4 to 15.

Embodiments of the invention will now be described by way of example only with reference to the accompanying drawings, of which:-
Figure 1 is a front view of a first embodiment of an ostomy appliance in accordance with the present invention;
Figure 2 is a side cross-sectional view of the ostomy appliance of figure 1;
Figure 3 is a further side cross-sectional view of the ostomy appliance of figure 1;
Figure 4 is a front view of a second embodiment of an ostomy appliance in accordance with the present invention;
Figure 5 is a side cross-sectional view of the ostomy appliance of figure 4;
Figure 6 is a further side cross-sectional view of the ostomy appliance of figure 4; and
Figure 7 is a perspective view of an example of a multi-layer filter for use in the first and second embodiments of ostomy appliance.

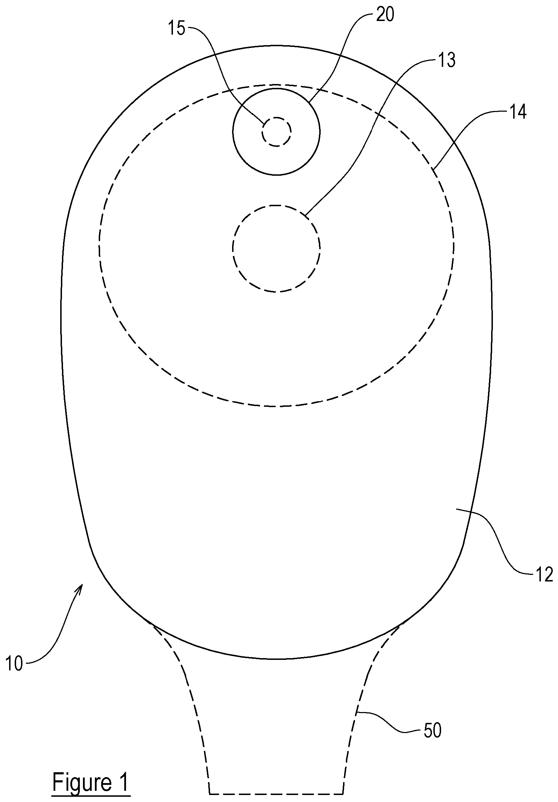

Referring firstly to figures 1 to 3 these show a first embodiment of an ostomy appliance in accordance with the present invention, shown generally at 10. The general construction of the ostomy appliance 10 is similar to those well known in the art and in that sense it includes first 11 and second 12 walls which are connected to each other at or near their peripheries, for example by welding or using an adhesive. The ostomy appliance 10 shown could be a drainable appliance, meaning that its contents could be emptied through an outlet (50, shown in broken lines) between the first 11 and second 12 walls.

The first wall 11 has a stoma-receiving opening 13 and is connected to a generally circular connection member 14 in the form of a flange for adhering the appliance 10 to a user around their stoma. The connection member 14 could be any appropriate shape, however.

The ostomy appliance 10 defines at least one chamber therein. A waste collecting chamber 30 is provided which communicates with the stoma-receiving opening 13 and, if the ostomy appliance is a drainable appliance, at its lower end with an outlet (50). The waste collecting chamber 30 is defined between the first 11 and second 12 walls and is provided as the primary chamber for collecting a user's waste, which enters the chamber through the opening 13 in the first wall 11. More chambers (not shown) may be provided within the ostomy appliance, for example in order to create a tortuous path for the waste gas to exit the appliance and/or to allow fluid in a further chamber to pass into the waste collecting chamber 30 via a non-return valve.

An aperture 15 is provided in the second wall 12 to permit waste gas within the ostomy appliance 10 to exit the appliance. The aperture 15 is preferably in use provided above the stoma-receiving opening 13 to reduce the likelihood of waste collected in the waste collecting chamber 30 from getting near to the aperture 15. The aperture 15 is preferably circular in shape but can be formed in a number of different shapes. The aperture 15 could in some embodiments of the present invention include a plurality of apertures in the form of perforations provided in the second wall 12. Although the aperture 15 is shown in figure 1 as being provided in the second wall 12, it may instead be provided in the first wall 11. Alternatively, apertures may be provided on both the first 11 and second 12 walls.

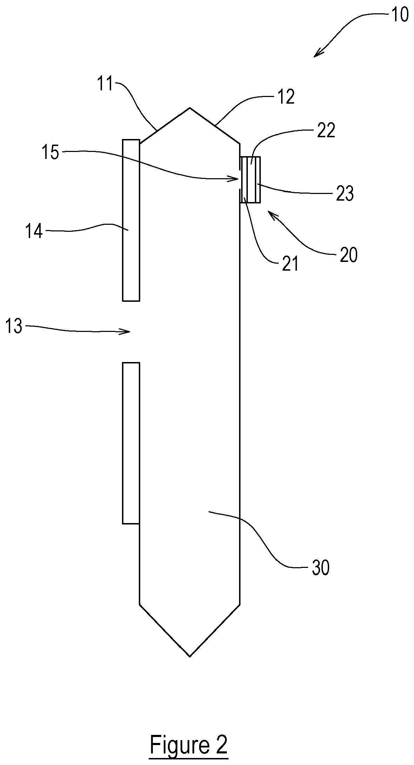

A filter 20 is provided externally of the ostomy appliance 10 as shown in figures 1 and 2. More specifically the filter 20 is attached to an exterior surface of the second wall 12. The filter 20 is attached to the second wall 12 adjacent the aperture 15 so that the aperture is fully covered by the filter 20. It can be seen on figure 1 that the aperture 15 is a smaller diameter than the diameter of the filter 20. Therefore, any waste gases that exit the ostomy appliance 10 through the aperture 15 must pass through the filter 20 in order to pass to atmosphere. The filter 20 is attached by an adhesive connection, although it could be connected in any appropriate way, for example heat welding.

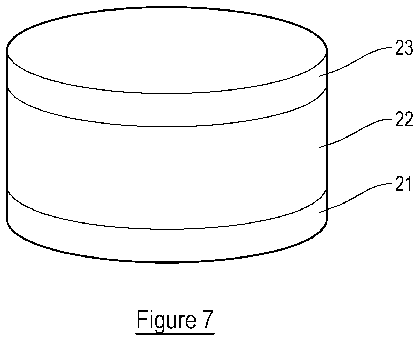

The filter 20 is a multi-layered filter as shown in more detail in figure 7. The multi-layered filter 20 preferably consists of three layers (although it could have more layers), including a hydrophobic layer 21, a de-odorising layer 22 and a gas and liquid impermeable layer 23. The multi-layered filter 20 is preferably constructed (but not limited to) in the order of hydrophobic layer 21, de-odorising layer 22 and gas and liquid impermeable layer 23.

The hydrophobic layer 21 is preferably Polytetrafluoroethylene (PTFE). The hydrophobic nature of PTFE ensures that water molecules are repelled from its surface, meaning that any interaction between the surface of the PTFE layer and water molecules is substantially resisted, and thus, the PTFE layer is waterproof. The hydrophobic layer 21 of the multi-layered filter 20 is advantageously positioned directly adjacent the second wall 12 so as to directly and completely cover the aperture 15 provided in the second wall 12. The hydrophobic layer 21 has a first side and second side with the first side connected directly to the second wall 12, said connection holding the multi-layer filter relative to the wall 12. The hydrophobic layer 21 is connected directly to the second wall 12 preferably by an adhesive, e.g. a holt melt adhesive or double-sided tape. Therefore, the hydrophobic layer 21 is in direct fluid communication with the waste collecting chamber 30 such that any waste gas exiting the ostomy appliance 10 must travel through the hydrophobic layer 21 first. The advantage of providing the hydrophobic layer 21 directly adjacent the aperture 15, and thus facing inwards towards the interior of the appliance 10, is that no liquid waste collected in the waste collecting chamber 30 can pass through the hydrophobic layer 21 and into contact with the other layers of the multi-layered filter 20. This reduces the risk of the filter, in particular the de-odorising layer 22, from getting damp and/or clogged. A damp and/or clogged filter can lead to ballooning of the ostomy appliance which can significantly reduce the effectiveness of the filter and the appliance. This will inevitably lead to the user having to replace the flawed ostomy appliance at a cost to the user.

Further to this, the multi-layered filter 20 is advantageously positioned on an external surface of the second wall 12 such that the de-odorising layer 22 and gas and liquid impermeable layer 23 cannot come into direct contact with the waste collected in the waste collecting chamber 30 which significantly reduces the risk of the filter 20 from getting damp and/or clogged. Further to this, providing a filter 20 externally of the ostomy appliance 10, e.g. external of the wall or walls which form the waste collecting chamber, allows for an easier and thus more cost effective manufacturing process as there is no need to place the filter carefully between layers of the ostomy appliance before they are connected together. Instead, the filter 20 can, if desired, be attached to the ostomy appliance once the walls of the appliance have been sealed together around its peripheries.

The de-odorising layer 22 is preferably a layer of carbon based de-odorising material that is positioned directly adjacent the hydrophobic layer 21 such that it lies on an opposite side of the hydrophobic layer 21 to the aperture 15 and wall 12. The de-odorising layer 22 receives waste gas travelling from the hydrophobic layer 21 and de-odorises the waste gas such that it is ready to exit the de-odorising layer 22 into atmosphere.

The gas and liquid impermeable layer 23 is preferably a layer of Cryovac^{™} material that does not allow gas or liquid to pass through its surface. The gas and liquid impermeable layer 23 is positioned directly adjacent the de-odorising layer 22 such that it lies on an opposite side of the de-odorising layer 22 to the hydrophobic layer 21. The gas and liquid impermeable layer 23 advantageously partially protects the de-odorising layer 22 from getting damp and/or clogged by moisture that is provided externally of the ostomy appliance, for example when a user is showering.

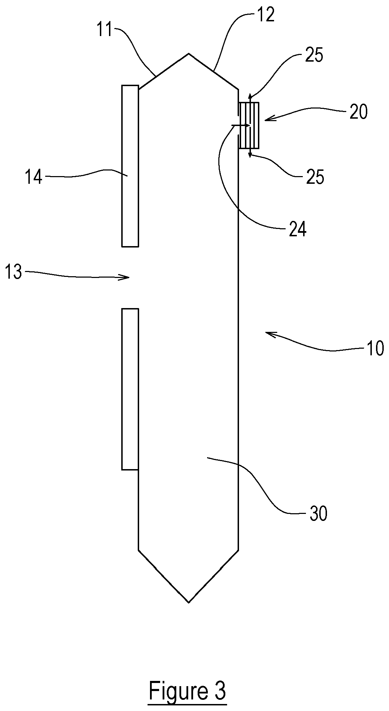

Referring to figure 3, arrows 24, 25 show the directional flow path taken by waste gas when exiting the waste collecting chamber 30 through the filter 20. Firstly, the waste gas travels through the aperture 15 and into the hydrophobic layer 21 in a direction substantially perpendicular to the second wall 12. Thereafter the waste gas continues to travel through the hydrophobic layer 21 and into the de-odorising layer 22 in a direction substantially perpendicular to the second wall 12. Finally, the waste gas travels through and exits the de-odorising layer 22 in a direction substantially parallel to the second wall 12, i.e. radially away from an axis of the filter 20. The positioning of the gas and liquid permeable layer 23 directly adjacent the de-odorising layer 21 ensures that all gas entering the filter passes through the de-odorising layer in a direction substantially parallel to the second wall 12.

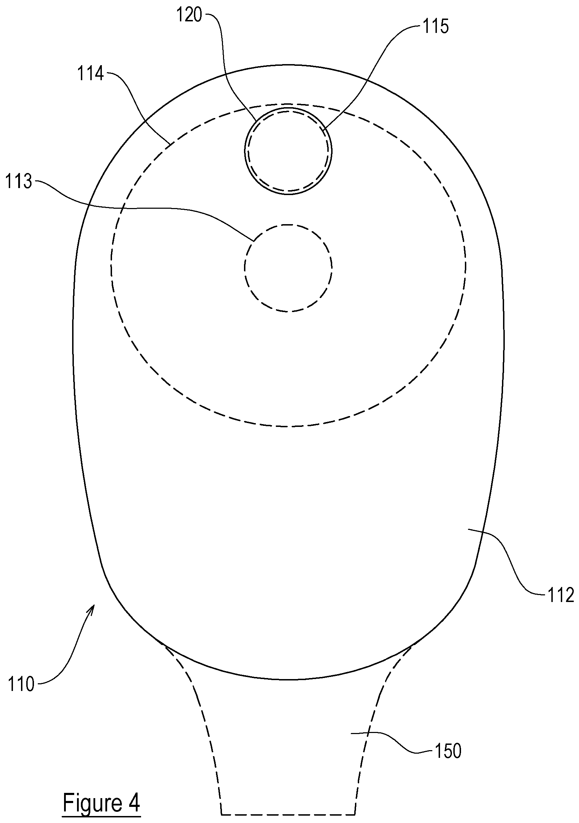

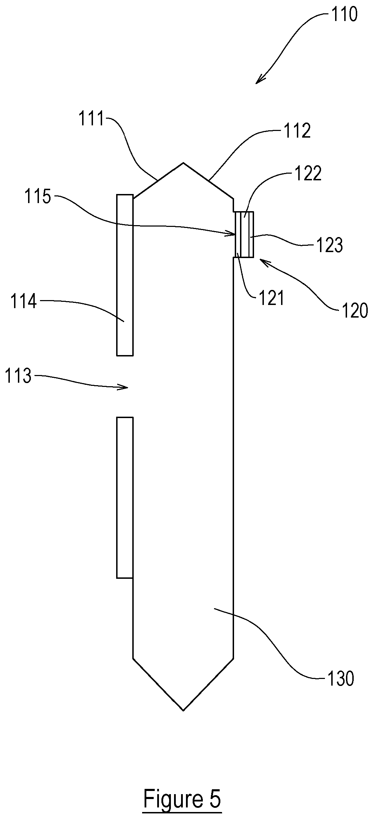

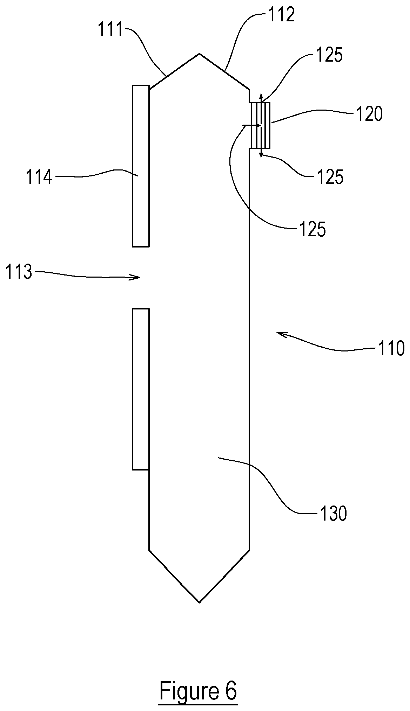

Figures 4 to 6 show a second embodiment of an ostomy appliance 110. Features which are in common with the first embodiment 10 have been given the same reference numeral with the addition of 100. Those features will not be discussed again here.

Referring to the second embodiment the main difference is the size of the aperture 115 in relation to the multi-layered filter 120. Instead of the aperture 115 being significantly smaller in diameter than the filter 120, the aperture 115 is substantially the same diameter as the filter 120. This provides for an increased area through which the waste gas can exit the ostomy appliance through the aperture 115 and into and through the hydrophobic layer 121 and further reduces the risk of the aperture 115 becoming completely blocked with waste. The flow through the aperture 115 and multi-layered filter 120 is substantially the same as that of the first embodiment, as shown by arrows 124, 125.

Whilst in the described embodiments the filter is shown as circular in front view, it should be appreciated that other shapes of filter could be used without departing from the scope of the present invention. In addition, whilst the filter described above has three layers, it should be appreciated that a two layer filter could be used, but also filters of four or more layers could be used.

When used in this specification and claims, the terms "comprises" and "comprising" and variations thereof mean that the specified features, steps or integers are included. The terms are not to be interpreted to exclude the presence of other features, steps or components.

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be utilised for realising the invention in diverse forms thereof within the limits of the scope of protection set out in the claims.

## Claims

1. An ostomy appliance comprising:
first and second walls connected to each other at or near their peripheries, the first wall having a stoma-receiving opening;
a connection member connected to the first wall for attaching the appliance to a user;
an aperture located in either of the first and/or second walls for permitting waste gases to exit the appliance;
a waste collecting chamber which communicates with the stoma-receiving opening and an additional chamber to create a tortuous path for waste gas to exit the appliance;
a multi-layer filter positioned adjacent and covering the aperture on an outwardly facing surface of the respective first and/or second wall,
wherein a layer of the filter is hydrophobic and wherein said hydrophobic layer is positioned directly adjacent the respective first and/or second wall, and
wherein the hydrophobic layer has a first side and a second side,
**characterised in that** the first side is connected directly to the respective first and/or second wall, said connection holding the multi-layer filter relative to the wall.

2. An ostomy appliance according to claim 1, wherein, in use, waste gas travels firstly through the aperture and into the hydrophobic layer.

3. An ostomy appliance comprising:
first and second walls connected to each other at or near their peripheries, the first wall having a stoma-receiving opening;
a connection member connected to the first wall for attaching the appliance to a user;
an aperture located in either of the first and/or second walls for permitting waste gases to exit the appliance;
a waste collecting chamber which communicates with the stoma-receiving opening and an additional chamber to create a tortuous path for waste gas to exit the appliance;
a multi-layer filter positioned adjacent and covering the aperture,
wherein a layer of the filter is hydrophobic and wherein said hydrophobic layer is positioned directly adjacent the respective first and/or second wall,
wherein, in use, waste gas travels firstly through the aperture and into the hydrophobic layer;
wherein the hydrophobic layer has a first side and a second side,
**characterised in that** the first side is connected directly to the respective first and/or second wall, said connection holding the multi-layer filter relative to the wall

4. An ostomy appliance according to claim 3, wherein the multi-layer filter is positioned adjacent and covering the aperture on an outwardly facing surface of the respective first and/or second wall.

5. An ostomy appliance according to any preceding claim, wherein the multi-layer filter is positioned adjacent and covering the aperture on an exterior surface of the respective first and/or second wall.

6. An ostomy appliance according to any preceding claim, wherein the hydrophobic layer is Polytetrafluoroethylene (PTFE).

7. An ostomy appliance according to any preceding claim, wherein the filter includes a de-odorising layer positioned adjacent the hydrophobic layer;
optionally wherein the de-odorising layer is preferably carbon based;
optionally wherein the filter includes a gas and liquid impermeable layer positioned adjacent the de-odorising layer;
preferably wherein the gas and liquid impermeable layer is preferably cryovac material.

8. An ostomy appliance according to claim 7, wherein the layers of the filter are arranged in the order of hydrophobic layer, de-odorising layer and gas and liquid impermeable layer;
optionally wherein the de-odorising layer is positioned directly adjacent the hydrophobic layer and wherein the gas and liquid impermeable layer is positioned directly adjacent the de-odorising layer.

9. An ostomy appliance according to any preceding claim, wherein waste gas travels through the aperture in a direction substantially perpendicular to the first and/or second wall; and/or
wherein waste gas travels through the hydrophobic layer in a direction substantially perpendicular to the first and/or second wall; and/or
wherein waste gas travels through the de-odorising layer in a direction substantially parallel to the first and/or second wall;
optionally wherein the gas and liquid impermeable layer is configured to ensure that all gas entering the filter passes through the de-odorising layer in a direction substantially parallel to the first and/or second wall.

10. An ostomy appliance according to any preceding claim, wherein the first side of the hydrophobic layer is heat welded directly to the respective first and/or second wall; and / or
wherein the first side of the hydrophobic layer is connected directly to the respective first and/or second wall by an adhesive, e.g. a hot melt adhesive or double-sided tape.

11. An ostomy appliance according to any preceding claim, wherein the first and second walls are connected to each other at their peripheries by welding.

12. An ostomy appliance according to any preceding claim, wherein the aperture is positioned in a top half of the appliance;
optionally wherein the aperture is positioned above the stoma-receiving opening;
optionally wherein the aperture is positioned directly above the stoma-receiving opening.

13. An ostomy appliance according to any preceding claim, including a plurality of apertures, wherein the multi-layer filter is positioned adjacent and covers each aperture;
optionally wherein the apertures are provided in the form of perforations in the second wall.

14. An ostomy appliance according to any preceding claim, wherein the aperture is smaller than the filter.

15. An ostomy appliance according to any preceding claim, wherein the waste collecting chamber is defined between the first and second walls; and/or
wherein the hydrophobic layer is in direct fluid communication with the waste collecting chamber; and/or
wherein the filter is provided external of the first wall and/or second wall which define the waste collecting chamber; and / or
wherein the ostomy appliance is a closed colostomy appliance; and / or
wherein the ostomy appliance is a drainable ostomy appliance.

## Patentansprüche

1. Stoma-Vorrichtung, die Folgendes umfasst:
eine erste und eine zweite Wand, die an oder nahe ihren Rändern miteinander verbunden sind, wobei die erste Wand eine Stoma-Aufnahmeöffnung aufweist;
ein mit der ersten Wand verbundenes Verbindungselement zum Anbringen der Vorrichtung an einem Benutzer;
eine Öffnung, die sich in der ersten und/oder der zweiten Wand befindet, um das Austreten von Ausscheidungsgasen aus der Vorrichtung zu ermöglichen;
eine Ausscheidungssammelkammer, die mit der Stoma-Aufnahmeöffnung und einer zusätzlichen Kammer in Verbindung steht, um einen gewundenen Weg für das Austreten von Ausscheidungsgas aus der Vorrichtung zu schaffen;
einen mehrschichtigen Filter, der neben der Öffnung positioniert ist und diese auf einer nach außen weisenden Fläche der jeweiligen ersten und/oder zweiten Wand abdeckt,
wobei eine Schicht des Filters hydrophob ist und wobei die genannte hydrophobe Schicht direkt neben der jeweiligen ersten und/oder zweiten Wand positioniert ist, und
wobei die hydrophobe Schicht eine erste Seite und eine zweite Seite hat,
**dadurch gekennzeichnet, dass** die erste Seite direkt mit der jeweiligen ersten und/oder zweiten Wand verbunden ist, wobei die genannte Verbindung den mehrschichtigen Filter relativ zur Wand hält.

2. Stoma-Vorrichtung nach Anspruch 1, wobei beim Gebrauch Ausscheidungsgas zunächst durch die Öffnung in die hydrophobe Schicht strömt.

3. Stoma-Vorrichtung, die Folgendes umfasst:
eine erste und eine zweite Wand, die an oder nahe ihren Rändern miteinander verbunden sind, wobei die erste Wand eine Stoma-Aufnahmeöffnung aufweist;
ein mit der ersten Wand verbundenes Verbindungselement zum Anbringen der Vorrichtung an einem Benutzer;
eine in der ersten und/oder zweiten Wand befindliche Öffnung, um das Austreten von Ausscheidungsgasen aus der Vorrichtung zu ermöglichen;
eine Ausscheidungssammelkammer, die mit der Stoma-Aufnahmeöffnung und einer zusätzlichen Kammer in Verbindung steht, um einen gewundenen Weg für das Austreten von Ausscheidungsgas aus der Vorrichtung zu erzeugen;
einen mehrschichtigen Filter, der neben der Öffnung positioniert ist und sie abdeckt,
wobei eine Schicht des Filters hydrophob ist und wobei die genannte hydrophobe Schicht direkt neben der jeweiligen ersten und/oder zweiten Wand positioniert ist,
wobei beim Gebrauch Ausscheidungsgas zunächst durch die Öffnung und in die hydrophobe Schicht strömt;
wobei die hydrophobe Schicht eine erste Seite und eine zweite Seite hat,
**dadurch gekennzeichnet, dass** die erste Seite direkt mit der jeweiligen ersten und/oder zweiten Wand verbunden ist, wobei die genannte Verbindung den mehrschichtigen Filter relativ zur Wand hält.

4. Stoma-Vorrichtung nach Anspruch 3, wobei der mehrschichtige Filter neben der Öffnung positioniert ist und diese auf einer nach außen weisenden Fläche der jeweiligen ersten und/oder zweiten Wand abdeckt.

5. Stoma-Vorrichtung nach einem vorherigen Anspruch, wobei der mehrschichtige Filter neben der Öffnung auf einer Außenfläche der jeweiligen ersten und/oder zweiten Wand positioniert ist und sie abdeckt.

6. Stoma-Vorrichtung nach einem vorherigen Anspruch, wobei die hydrophobe Schicht aus Polytetrafluorethylen (PTFE) besteht.

7. Stoma-Vorrichtung nach einem vorherigen Anspruch, wobei der Filter eine Desodorierungsschicht umfasst, die neben der hydrophoben Schicht positioniert ist;
wobei optional die Desodorierungsschicht vorzugsweise auf Kohlenstoff basiert;
wobei optional der Filter eine gas- und flüssigkeitsundurchlässige Schicht umfasst, die neben der Desodorierungsschicht positioniert ist;
wobei vorzugsweise die gas- und flüssigkeitsundurchlässige Schicht vorzugsweise aus Cryovac-Material besteht.

8. Stoma-Vorrichtung nach Anspruch 7, wobei die Schichten des Filters in der Reihenfolge hydrophobe Schicht, Desodorierungsschicht und gas- und flüssigkeitsundurchlässige Schicht angeordnet sind;
wobei optional die Desodorierungsschicht direkt neben der hydrophoben Schicht positioniert ist und wobei die gas- und flüssigkeitsundurchlässige Schicht direkt neben der Desodorierungsschicht positioniert ist.

9. Stoma-Vorrichtung nach einem vorherigen Anspruch, wobei Ausscheidungsgas durch die Öffnung in einer Richtung im Wesentlichen lotrecht zur ersten und/oder zweiten Wand strömt; und/oder
wobei Ausscheidungsgas durch die hydrophobe Schicht in einer Richtung im Wesentlichen lotrecht zur ersten und/oder zweiten Wand strömt; und/oder
wobei Ausscheidungsgas durch die Desodorierungsschicht in einer Richtung im Wesentlichen parallel zur ersten und/oder zweiten Wand strömt;
wobei optional die gas- und flüssigkeitsundurchlässige Schicht konfiguriert ist zum Sicherstellen, dass sämtliches in den Filter eintretendes Gas in einer Richtung im Wesentlichen parallel zur ersten und/oder zweiten Wand durch die Desodorierungsschicht strömt.

10. Stoma-Vorrichtung nach einem vorherigen Anspruch, wobei die erste Seite der hydrophoben Schicht direkt mit der jeweiligen ersten und/oder zweiten Wand wärmeverschweißt ist; und/oder
wobei die erste Seite der hydrophoben Schicht direkt mit der jeweiligen ersten und/oder zweiten Wand mit einem Klebstoff, z. B. einem Schmelzklebstoff oder einem doppelseitigen Klebeband, verbunden ist.

11. Stoma-Vorrichtung nach einem vorherigen Anspruch, wobei die erste und die zweite Wand an ihren Rändern durch Schweißen miteinander verbunden sind.

12. Stoma-Vorrichtung nach einem vorherigen Anspruch, wobei die Öffnung in einer oberen Hälfte der Vorrichtung positioniert ist;
wobei optional die Öffnung oberhalb der Stoma-Aufnahmeöffnung positioniert ist;
wobei optional die Öffnung direkt oberhalb der Stoma-Aufnahmeöffnung positioniert ist.

13. Stoma-Vorrichtung nach einem vorherigen Anspruch, die mehrere Öffnungen aufweist, wobei der mehrschichtige Filter neben jeder Öffnung positioniert ist und sie abdeckt;
wobei die Öffnungen optional in Form von Perforationen in der zweiten Wand vorgesehen sind.

14. Stoma-Vorrichtung nach einem vorherigen Anspruch, wobei die Öffnung kleiner ist als der Filter.

15. Stoma-Vorrichtung nach einem vorherigen Anspruch, wobei die Ausscheidungssammelkammer zwischen der ersten und der zweiten Wand definiert ist; und/oder
wobei die hydrophobe Schicht in direkter Fluidverbindung mit der Ausscheidungssammelkammer steht; und/oder
wobei der Filter außerhalb der die Ausscheidungssammelkammer begrenzenden ersten Wand und/oder zweiten Wand vorgesehen ist; und/oder
wobei die Stoma-Vorrichtung eine geschlossene Kolostomie-Vorrichtung ist; und/oder
wobei die Stoma-Vorrichtung eine entleerbare Stoma-Vorrichtung ist.

## Revendications

1. Appareil de stomie comprenant :
une première paroi et une deuxième paroi reliées l'une à l'autre au niveau ou à proximité de leurs périphéries, la première paroi comportant une ouverture de réception de stomie ;
un élément de connexion relié à la première paroi pour fixer l'appareil à un utilisateur ;
un orifice situé dans la première et/ou la deuxième paroi pour permettre à des gaz provenant des déchets de sortir de l'appareil ;
une chambre de collecte des déchets qui communique avec l'ouverture de réception de stomie et avec une chambre supplémentaire pour créer un trajet tortueux permettant aux gaz provenant des déchets de sortir de l'appareil ;
un filtre multicouche positionné de façon adjacente à l'orifice et recouvrant celui-ci sur une surface orientée vers l'extérieur de la première paroi et/ou de la deuxième paroi respective(s),
dans lequel une couche du filtre est hydrophobe et dans lequel ladite couche hydrophobe est positionnée de façon directement adjacente à la première paroi et/ou à la deuxième paroi respective(s), et
dans lequel la couche hydrophobe comporte un premier côté et un deuxième côté,
**caractérisé en ce que** le premier côté est relié directement à la première paroi et/ou à la deuxième paroi respective(s), ladite connexion maintenant le filtre multicouche en place par rapport à la paroi.

2. Appareil de stomie selon la revendication 1, dans lequel, en cours d'utilisation, les gaz provenant des déchets traversent d'abord l'orifice et pénètrent dans la couche hydrophobe.

3. Appareil de stomie comprenant :
une première paroi et une deuxième paroi reliées l'une à l'autre au niveau ou à proximité de leurs périphéries, la première paroi comportant une ouverture de réception de stomie ;
un élément de connexion relié à la première paroi pour fixer l'appareil à un utilisateur ;
un orifice situé dans la première paroi et/ou la deuxième paroi pour permettre à des gaz provenant des déchets de sortir de l'appareil ;
une chambre de collecte des déchets qui communique avec l'ouverture de réception de stomie et avec une chambre supplémentaire pour créer un trajet tortueux permettant aux gaz provenant des déchets de sortir de l'appareil ;
un filtre multicouche positionné de façon adjacente à l'orifice et recouvrant celui-ci,
dans lequel une couche du filtre est hydrophobe et dans lequel ladite couche hydrophobe est positionnée de façon directement adjacente à la première paroi et/ou à la deuxième paroi respective(s), et
dans lequel, en cours d'utilisation, les gaz provenant des déchets traversent d'abord l'orifice et pénètrent dans la couche hydrophobe.
dans lequel la couche hydrophobe comporte un premier côté et un deuxième côté,
**caractérisé en ce que** le premier côté est relié directement à la première paroi et/ou à la deuxième paroi respective(s), ladite connexion maintenant le filtre multicouche en place par rapport à la paroi.

4. Appareil de stomie selon la revendication 3, dans lequel le filtre multicouche est positionné de façon adjacente à l'orifice et recouvre celui-ci sur une surface orientée vers l'extérieur de la première paroi et/ou de la deuxième paroi respective(s).

5. Appareil de stomie selon l'une quelconque des revendications précédentes, dans lequel le filtre multicouche est positionné de façon adjacente à l'orifice et recouvre celui-ci sur une surface extérieure de la première paroi et/ou de la deuxième paroi respective(s).

6. Appareil de stomie selon l'une quelconque des revendications précédentes, dans lequel la couche hydrophobe est en polytétrafluoroéthylène (PTFE).

7. Appareil de stomie selon l'une quelconque des revendications précédentes, dans lequel le filtre comprend une couche désodorisante positionnée de façon adjacente à la couche hydrophobe ;
optionnellement dans lequel la couche désodorisante est de préférence à base de carbone ;
optionnellement dans lequel le filtre comprend une couche imperméable aux gaz et aux liquides positionnée de façon adjacente à la couche désodorisante ;
de préférence dans lequel la couche imperméable aux gaz et aux liquides est de préférence constituée d'un matériau Cryovac.

8. Appareil de stomie selon la revendication 7, dans lequel les couches du filtre sont disposées dans l'ordre suivant : couche hydrophobe, couche désodorisante, et couche imperméable aux gaz et aux liquides ;
optionnellement dans lequel la couche désodorisante est positionnée de façon directement adjacente à la couche hydrophobe et dans lequel la couche imperméable aux gaz et aux liquides est positionnée de façon directement adjacente à la couche désodorisante.

9. Appareil de stomie selon l'une quelconque des revendications précédentes, dans lequel les gaz provenant des déchets traversent l'orifice dans une direction sensiblement perpendiculaire à la première paroi et/ou à la deuxième paroi ; et/ou
dans lequel les gaz provenant des déchets traversent la couche hydrophobe dans une direction sensiblement perpendiculaire à la première paroi et/ou à la deuxième paroi ; et/ou
dans lequel les gaz provenant des déchets traversent la couche désodorisante dans une direction sensiblement parallèle à la première paroi et/ou à la deuxième paroi ;
optionnellement dans lequel la couche imperméable aux gaz et aux liquides est configurée pour garantir que tous les gaz pénétrant dans le filtre traversent la couche désodorisante dans une direction sensiblement parallèle à la première paroi et/ou à la deuxième paroi.

10. Appareil de stomie selon l'une quelconque des revendications précédentes, dans lequel le premier côté de la couche hydrophobe est thermosoudé directement à la première paroi et/ou à la deuxième paroi respective(s) ; et/ou
dans lequel le premier côté de la couche hydrophobe est relié directement à la première paroi et/ou à la deuxième paroi respective(s) au moyen d'un adhésif, par exemple d'un adhésif thermofusible ou d'un ruban adhésif double face.

11. Appareil de stomie selon l'une quelconque des revendications précédentes, dans lequel la première paroi et la deuxième paroi sont reliées l'une à l'autre au niveau de leurs périphéries par soudage.

12. Appareil de stomie selon l'une quelconque des revendications précédentes, dans lequel l'orifice est positionné dans une moitié supérieure de l'appareil ;
optionnellement dans lequel l'orifice est positionné au-dessus de l'ouverture de réception de stomie ;
optionnellement dans lequel l'orifice est positionné directement au-dessus de l'ouverture de réception de stomie ;

13. Appareil de stomie selon l'une quelconque des revendications précédentes, comprenant une pluralité d'orifices, dans lequel le filtre multicouche est positionné de façon adjacente à chaque orifice et recouvre chacun d'eux ;
optionnellement dans lequel les orifices sont présents sous la forme de perforations dans la deuxième paroi.

14. Appareil de stomie selon l'une quelconque des revendications précédentes, dans lequel l'orifice est plus petit que le filtre.

15. Appareil de stomie selon l'une quelconque des revendications précédentes, dans lequel la chambre de collecte des déchets est délimitée entre la première paroi et la deuxième paroi ; et/ou
dans lequel la couche hydrophobe est en communication fluidique directement avec la chambre de collecte des déchets ; et/ou
dans lequel le filtre est présent à l'extérieur de la première paroi et/ou de la deuxième paroi délimitant la chambre de collecte des déchets ; et/ou
l'appareil de stomie étant un appareil de colostomie fermé ; et/ou
l'appareil de stomie étant un appareil de colostomie vidangeable.
